# EUROPÄISCHE PATENTANMELDUNG

(11) **EP 1 064 939 A2**
(43) Veröffentlichungstag der Anmeldung: **03.01.2001**
(21) Anmeldenummer: 00113069.9
(22) Anmeldetag: 26.06.2000
(51) Int. Cl.: A61K 9/70, A61P 1/08

(54) **Transdermalsysteme zur Abgabe von 5-HT3-Rezeptor-Antagonisten und ihre Verwendung zur antiemetischen Behandlung**

(30) Priorität: 25.06.1999 DE 19929197
(71) Anmelder: Novosis Pharma AG, 80807 München (DE)
(72) Erfinder: Fischer, Wilfried, Dr., 80807 München (DE); Stierle, Axel, 80807 München (DE)
(74) Vertreter: Biagosch, Stephan (DE)

(57) **Zusammenfassung**

Die Erfindung betrifft ein Transdermalsystem zur Abgabe von 5-HT₃-Rezeptor-Antagonisten, das dadurch gekennzeichnet ist, daß es eine Zusammensetzung aus einem 5-HT₃-Rezeptor-Antagonisten als Wirkstoff, Teebaumöl, einem mit Wasser mischbaren Lösungsmittel und Wasser enthält.

## Beschreibung

Die Erfindung betrifft Transdermalsysteme zur Abgabe von 5-HT₃-Rezeptor-Antagonisten und ihre Verwendung zur antiemetischen Behandlung.

### Einleitung und Stand der Technik

Die Cyctostatika-Therapie von Tumorerkrankungen, z.B. mit Cisplatin-Derivaten u.a., ist häufig von starkem Übelkeitsempfinden begleitet und auch die Tumore selbst können Übelkeit (Nausea) oder Erbrechen (Emesis) hervorrufen. Die moderne Behandlung starker Emesis erfolgt derzeit mit Antagonisten der Serotonin(= 5-Hydroxytryptamin = 5-HT)-Rezeptoren des Typs 5-HT₃.

Als Beispiel für einen spezifischen Antagonisten, sowohl der Chemorezeptoren als auch von Rezeptoren des Gastrointestinaltraktes, sei Ondansetron-Hydrochlorid genannt. Weitere 5-HT₃-Antagonisten sind Granisetron-Hydrochlorid, Azasetron-Hydrochlorid, Ramosetron-Hydrochlorid oder deren Basen.

Im Plasma treten ungefähr 1 Stunde nach oraler Gabe und 6 - 20 Minuten nach einer intravenösen Gabe von Ondansetron-Hydrochlorid die Spitzenkonzentrationen auf. Die mittlere Eliminationshalbwertzeit liegt bei gesunden Probanden bei 3,5 Stunden und ist bei älteren Patienten auf ca. 7,9 Stunden verlängert.

Ondansetron wirkt zuverlässig bei der Behandlung der Nausea und der Emesis bei Patienten, die sich einer Behandlung mit Cytostatika, wie Cisplatin-Derivaten, unterziehen müssen.

Als Nebenwirkungen treten Kopfschmerz, Diarrhöe und vorübergehende Abnormitäten in Leberfunktionstests auf. Die Dosis an Ondansetron zur Prophylaxe von durch Cytostatika induziertem Erbrechen beträgt im allgemeinen 0.15 mg/kg i.v. dreimal täglich alle 4 Stunden.

Chemisch ist Ondansetron das Dihydrat des (±)-1,2,3,9-Tetrahydro-9-methyl-3-[(2-methyl-1H-imidazol-1-yl)methyl]-4H-carbazol-4-on-Monohydrochlorids.

Die Summenformel lautet C₁₈H₁₉N₃0 · HCI · 2H₂0, was einer Molekularmasse von 365.9 g entspricht.

Ondansetron wird im Menschen zwar stark metabolisiert, jedoch finden sich nur etwa 5 % einer radioaktiv markierten Dosis im Urin wieder. Der primäre Metabolisierungsweg ist die Hydroxylierung am Indolring, gefolgt von der Glucuronidierung oder Sulfatierung.

In gesunden Probanden wurden die in der Tabelle zusammengestellten pharmakokinetischen Daten nach intravenöser Einmalgabe einer Dosis von 0.15-mg/kg i.v. ermittelt.

| **Altersgruppe (Jahre)** | **Plasma-Spitzenkonzentration (ng/ml** | **Mittlere Halbwertszeit (h)** | **Plasma-Clearance (l/h/kg)** |
|---|---|---|---|
| 19-40 | 11 102 | 3,5 | 0,38 |
| 61-74 | 12 106 | 4,7 | 0,32 |
| ≥ 75 | 11 170 | 5,5 | 0,26 |

Die Plasmaprotein-Bindung des Ondansetrons beträgt ca. 70% bis 76%, und zwar in vitro im pharmakologisch wichtigen Konzentrationsbereich von 10 - 500 ng/ml gemessen.

Nach oraler Verabreichung wird Ondansetron gut absorbiert und unterliegt einer First-Pass-Metabolisierung bei der Leberpassage. Nach der Applikation von 8-mg-Tabletten an gesunde Probanden beträgt die relative Bioverfügbarkeit ca. 56%

Da die Applikation von 5-HT₃-Antagonisten vor der Cytostatika-Verabreichung durch eine parenteralen Infusion erfolgt, bei oraler Gabe die Bioverfügbarkeit nur ca. 50% (z.B. Ondansetron) beträgt und eine Langzeitapplikation der Substanzen erforderlich ist, muß für die Patienten, die gerade bei der Cytostatika-Therapie durch Injektionen und Infusionen stark belastet sind, eine angenehmere und zuverlässige Applikationsart zur Verfügung gestellt werden.

Die transdermale Verabreichung von hochwirksamen Substanzen, wie-Hormonen, starken Analgetika, Nitraten etc., ist seit einigen Jahren bekannt

Ondansetron wird unter dem Handelsnamen Zofran® in zur Injektion geeigneter Form oder in Form von Tabletten zur oralen Verabreichung vertrieben. Eine zur transdermalen Applikation geeignete Form existiert aber derzeit nicht.

Die WO 9825592, basierend auf der JP 96-346460, beschreibt Transdermalsysteme, die aus mindestens 3 Schichten bestehen: (A) einer für den Wirkstoff impermeablen "Backing-Layer", (B) einer als Wirkstoffreservoir dienenden Schicht, die einen Serotonin-Rezeptor-Antagonisten enthält, und (C) einer die Wirkstofffreisetzung kontrollierenden Schicht. Diese Schicht besteht aus einem druckempfindlichen Haftklebstoff, der die Wirkstofffreisetzung kontrolliert.

Die WO 9407468, basierend auf der US 92-956635, der Fa. Cygnus Therapeutic Systems, USA, beschreibt als Transdermalsystem eine "Two-phase matrix for sustained-release transdermal pharmaceuticals". Der Kern dieser Erfindung besteht darin, daß in einem hydrophoben kontinuierlichen Polymer, das gegebenenfalls ein als Permeationsförderer wirkendes hydrophobes Lösemittel enthält, eine zweite Phase dispergiert wird, die aus einem hydratisierten anorganischen Silikat, in dessen adsorbierter Wasserphase ein wasserlöslicher Wirkstoff gelöst ist, besteht. So kann beispielsweise eine Matrix, die aus 4% (R)-(-)-N-(1-methyl-4-(3-methylbenzyl)hexahydro-1H-1,4-diazepin-6-yl)-1H-indazol-3-carboxamid · 2HCl, 10% Propylenglycolmonolaureat, 20% Propylenglycol, 20% Wasser, 7% Calciumsilicat, 2% Pluronic L-121 und Dimethylsiloxan-Adhesiv ad 100% besteht, hergestellt werden, indem eine Lösung der o.g. Zusammensetzung in einer Dicke von 250 µm auf eine Polyesterfolie aufgetragen, getrocknet und mit einer Polyesterfolie kaschiert wird. Der Fluß des Wirkstoffes durch die humane Epidermis in einer Diffusionszelle betrug in vitro 17,1 µg/cm²/h, verglichen mit 0,8 µg/cm²/h einer zum Vergleich benutzten Klebstoffmatrix aus Styrol-Butadien-Copolymer.

Transdermalsysteme (was im weiteren Sinn zu verstehen ist, d. h. Systeme, die allgemein zur Aufnahme durch die Haut geeignet sind) für 5-HT₃-Rezeptor-Antagonisten gibt es bisher aber nicht.

Aufgabe der Erfindung ist daher die Bereitstellung eines zuverlässig wirkenden und hautverträglichen Transdermalsystems zur Abgabe von 5-HT₃-Rezeptor-Antagonisten, mit dem sich die dem Stand der Technik anhaftenden Nachteile vermeiden lassen.

Diese Aufgabe wird erfindungsgemäß mit einem Transdermalsystem zur Abgabe von 5-HT₃-Rezeptor-Antagonisten mit den in Anspruch 1 angegebenen Merkmalen gelöst.

Weitere vorteilhafte Ausführungsformen sind Gegenstand der Unteransprüche.

Die Erfindung gibt ferner die Verwendung der erfindungsgemäßen Transdermalsysteme zur Abgabe von 5-HT₃-Rezeptor-Antagonisten zur antiemetischen Behandlung (d. h. zur Behandlung von Nausea und Emesis) an.

### Detaillierte Beschreibung der Erfindung

Das erfindungsgemäße Transdermalsystem gestattet eine bessere Permeation von Ondansetron durch die Haut durch die Verwendung einer Kombination aus Teebaumöl, einem mit Wasser mischbaren Lösemittel und Wasser als Resorptions- bzw. Permeationsförderer.

Es wurde überraschenderweise in Versuchen zur Verbesserung der Resorption gefunden, daß der üblicherweise als Resorptionsförderer, z.B. bei Sexualhormonen und Analgetika wie Fentanyl, verwendete Alkohol bei Ondansetron keine ausreichende Verbesserung der Resorption ergibt. Ferner war überraschend, daß das ionische Hydrochlorid in Kombination mit dem beschriebenen Lösemittelgemisch besser permeiert als Ondansetron in Form der freien, nichtionischen Base.

Nachstehend wird die Erfindung durch Abbildungen näher erläutert. Es zeigen
Abb. 1 Ondansetron-Permeationen aus Ethanol bzw. Teebaumöl und
Abb. 2 Ondansetron-Permeationen aus Wasser.

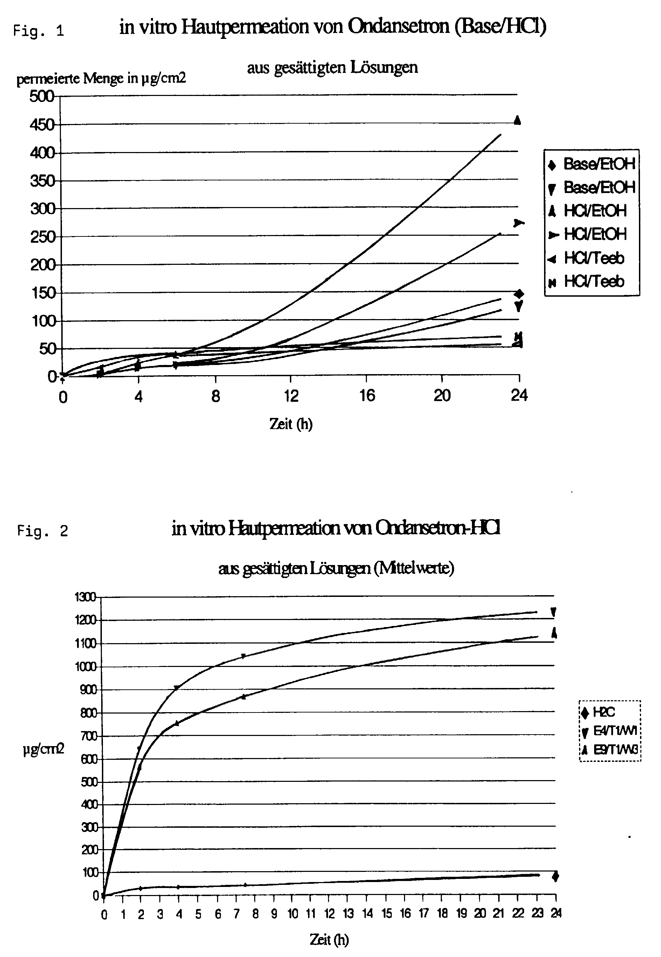

In Abb. 1 ist das Ausmaß der Permeation von Ondansetron (in Form der freien Base bzw. des Hydrochlorids) durch die Haut in vitro unter Verwendung von jeweils gesättigten Lösungen von Ondansetron-HCI bzw. der freien Base in Ethanol bzw. des Hydrochlorides in Teebaumöl dargestellt. Gesättigte Lösungen wurden verwendet, um jeweils maximale thermodynamische Aktivitäten zu erzielen.

Abb. 1 zeigt, daß sich die beste Permeation durch die Haut bei Verwendung des Hydrochlorides in Ethanol ergibt. Teebaumöl selbst ergibt eine unzureichende Permeation, während die Base in Ethanol nur leicht verbesserte Ergebnisse liefert.

Appliziert man, wie in Abb. 2 dargestellt, eine gesättigte wäßrige Lösung von Ondansetron-Hydrochlorid auf Nacktmaushaut, unterscheiden sich die Ergebnisse nicht von denen, die unter Verwendung von reinem Teebaumöl erhalten werden.

Wird dagegen eine Lösung von Ondansetron-Hydrochlorid in einem Gemisch bzw. einer Zusammensetzung aus Ethanol, Teebaumöl und Wasser auf die Haut appliziert, so verändert sich das Permeationsverhalten des Wirkstoffes unerwartet. In der ersten 4 - 5 Stunden läßt sich ein extrem starker Anstieg des Wirkstoffflußes beobachten. Danach flacht, vermutlich wegen der Abnahme der Wirkstoffkonzentration im Donorkompartiment, die Permeationskurve ab. Nach 24 Stunden sind ca 1,2 mg Ondansetron-Hydrochlorid pro cm² permeiert, d. h. die ca. 12fache Menge, die aus einer gesättigten wäßrigen Lösung durch die Maushaut diffundiert.

Die erfindungsgemäßen Lösungen mit 5-HT₃-Antagonisten als Wirkstoff können in gel-, salben- oder cremeartige Trägersysteme (Transdermalsysteme im weiteren Sinn) eingearbeitet und in flächigen Transdermalsystemen im engeren Sinn, d. h. sog. "transdermalen therapeutischen Systemen (TTS)", z.B. Wirkstoffpflastern, oder in Reservoirsystemen (Reservoir-TTS) verwendet werden.

Bevorzugte Ausführungsformen sind Transdermalsysteme im engeren Sinn, insbesondere solche mit Reservoir. Diese Transdermalsysteme bestehen aus einer wirkstoffundurchlässigen Abdeckung bzw. Deckfolie (Backing-Foil), die aus Polyester, Polypropylen, Polyethylen o.ä. besteht. Die Backing-Foil bildet zusammen mit einer mikroporösen Membran, die den Wirkstoffdurchtritt kontrollieren kann oder keine Diffsionskontrolle ausübt, ein beutelförmiges Reservoir, in das die erfindungsgemäßen Wirkstofflösungen eingefüllt werden. Nach dem Einfüllen wird die Backing-Foil mit der Membran verschweißt oder verklebt und anschließend gegebenenfalls eine Klebstoffschicht aufgebracht, die das Transdermalsystem auf der Haut fixiert. Statt der Klebstoffschicht kann auch ein Klebstoffring angebracht werden, so daß die Membran direkt auf der Haut aufliegt.

Im folgenden wird die Erfindung anhand von Beispielen erläutert.

### Beispiel 1

### Wirkstofflösung

In einem Gemisch aus 1 Teil Wasser, 1 Teil Teebaumöl und 4 Teilen Ethanol wird Ondansetron-Hydrochlorid bis zur Sättigung gelöst.

### Reservoir-TTS

Mit einem geeigneten thermischen Schweißgerät wird eine heißsiegelfähig beschichtete Polyesterfolie mit einer Stärke von 19 µm und einer kreisförmigen Fläche von 30 cm² mit einer Celluloseacetatmembran mit einer Porenweite von ca. 0,1 µm am Rand so verschweißt, daß eine Einfüllöffnung verbleibt, durch die 1 ml der o.g. Wirkstofflösung in den entstandenen Beutel eingefüllt wird. Anschließend wird die Einfüllöffnung ebenfalls verschweißt. Es entsteht eine kreisförmige Fläche von 20 cm², durch die membranseitig die Wirkstofflösung herausdiffundieren kann. Zur Fixierung auf der Haut wird über den Rand des Flüssigkeitsreservoirs ein Klebstoffring montiert, der aus einem druckempfindlichen Haftklebstoff, z.B. Duro-Tak 386-2287 (Fa. National, NL-Zutphen), auf einer 19 µm starken Polyesterfolie, z.B. Hostaphan MN 19 (Mitsubishi Foils, D-Frankfurt), besteht. Das gesamte System wird auf eine silikonisierte Polyesterfolie oder silikonisiertes Papier (Release-Liner) montiert. Der Release-Liner wird vor der Applikation auf die Haut entfernt und verworfen.

### Beispiel 2

### Wirkstofflösung

In einem Gemisch aus 3 Teilen Wasser, 1 Teil Teebaumöl und 9 Teilen Ethanol wird Ondansetron-Hydrochlorid bis zur Sättigung gelöst.

### Reservoir-TTS

Eine silikonisierte Polyesterfolie wird mit einer Rakel gleichmäßig mit einer Lösung eines druckempfindlichen Acrylat-Haftklebstoffs (z.B. ETA 1, Fa. Adhesives Research, Glen Rock, USA) beschichtet, worauf das Lösemittel abdampft wird, so daß eine gleichmäßige Schicht mit einer Dicke von 40 µm entsteht. Auf die Klebstoffschicht wird eine Polypropylenmembran mit einer mittleren Porenweite von 0,2 µm aufkaschiert. Dann wird mit einem geeigneten thermischen Schweißgerät eine heißsiegelfähig beschichtete kreisförmige Polyesterfolie mit einer Stärke von 19 µm und einer Fläche von 20 cm² mit der Membranseite des o.g. Laminats am Rand so verschweißt, daß eine Einfüllöffnung verbleibt, durch die 0,5 ml der o.g. Wirkstofflösung in den entstandenen Beute eingefüllt werden. Anschließend wird die Einfüllöffnung ebenfalls verschweißt. Es entsteht eine kreisförmige Fläche von 10 cm². Nach dem Abziehen der silikonisierten Polyesterfolie kann das Transdermalsystem mit der Klebstoffschicht auf die Haut geklebt werden. Die Wirkstoffabgabe an den Körper erfolgt durch die mikroporöse Membran und die sich anschließende Klebstoffschicht.

## Patentansprüche

1. Transdermalsystem zur Abgabe von 5-HT₃-Rezeptor-Antagonisten, dadurch gekennzeichnet, daß es eine Zusammensetzung aus einem 5-HT₃-Rezeptor-Antagonisten als Wirkstoff, Teebaumöl, einem mit Wasser mischbaren Lösungsmittel und Wasser enthält.

2. Transdermalsystem nach Anspruch 1, dadurch gekennzeichnet, daß es sich bei dem 5-HT₃-Rezeptor-Antagonisten um Ondansetron-Hydrochlorid, Granisetron-Hydrochlorid, Azasetron-Hydrochlorid, Ramosetron-Hydrochlorid oder deren Basen handelt.

3. Transdermalsystem nach einem der vorherigen Ansprüche, dadurch gekennzeichnet, daß es sich bei dem mit Wasser mischbaren Lösungsmittel um Ethanol handelt.

4. Transdermalsystem nach einem der Ansprüche 1 bis 3, dadurch gekennzeichnet, daß es sich um eine Zusammensetzung aus 1 Teil Wasser, 1 Teil Teebaumöl und 4 Teilen Ethanol handelt, in der Ondansetron-Hydrochlorid bis zur Sättigung gelöst ist.

5. Transdermalsystem nach einem der Ansprüche 1 bis 3, dadurch gekennzeichnet, daß es sich um eine Zusammensetzung aus 3 Teilen Wasser, 1 Teil Teebaumöl und 9 Teilen Ethanol handelt, in der Ondansetron-Hydrochlorid bis zur Sättigung gelöst ist.

6. Transdermalsystem nach einem der vorherigen Ansprüche, dadurch gekennzeichnet, daß die Zusammensetzung außerdem einen zur Herstellung von Gelen, Salben oder Cremes geeigneten pharmazeutisch akzeptablen Träger enthält.

7. Transdermalsystem nach einem der Ansprüche 1 bis 5, dadurch gekennzeichnet, daß die Zusammensetzung aus einem 5-HT₃-Rezeptor-Antagonisten als Wirkstoff, Teebaumöl, einem mit wasser mischbaren Lösungsmittel und Wasser in einer Schicht eines flächigen selbstklebenden Pflasters mit mehrschichtigem Aufbau enthalten ist.

8. Transdermalsystem nach Anspruch 7, dadurch gekennzeichnet, daß neben der die Wirkstoffzusammensetzung enthaltenden Schicht eine Abdeckung, eine Klebstoffschicht und auf der der Abdeckung gegenüberliegenden Seite ein die Klebstoffschicht temporär abdeckender und abziehbarer Träger vorgesehen sind.

9. Transdermalsystem nach Anspruch 8, dadurch gekennzeichnet, daß die Wirkstoffzusammensetzung in der Klebstoffschicht enthalten ist.

10. Transdermalsystem nach Anspruch 8 oder 9, dadurch gekennzeichnet, daß die Abdeckung aus Kunststoffolie, Gewebe oder Vlies besteht.

11. Transdermalsystem nach einem der Ansprüche 8 bis 10, dadurch gekennzeichnet, daß der Träger aus Kunststoffolie, Papier oder einem Laminat daraus besteht.

12. Transdermalsystem nach einem der Ansprüche 1 bis 5, dadurch gekennzeichnet, daß die Zusammensetzung aus einem 5-HT₃-Rezeptor-Antagonisten als Wirkstoff, Teebaumöl, einem mit Wasser mischbaren Lösungsmittel und Wasser in einem von einer mikroporösen, wirkstoffdurchlässigen Membran gebildeten Reservoir eines flächigen selbstklebenden Pflasters enthalten ist.

13. Transdermalsystem nach Anspruch 12, dadurch gekennzeichnet, daß die wirkstoffdurchlässige Membran aus Celluloseacetat, Polyester oder Polypropylen besteht.

14. Transdermalsystem nach Anspruch 12 oder 13, dadurch gekennzeichnet, daß die wirkstoffdurchlässige Membran eine Porengröße von 0,1 bis 0,2 µm hat.

15. Transdermalsystem nach einem der Ansprüche 12 bis 14, dadurch gekennzeichnet, daß neben dem die Wirkstoffzusammensetzung enthaltenden Reservoir eine wirkstoffundurchlässige Abdeckung, ein das Reservoir umgebender Klebstoffring und ein auf der der Abdeckung gegenüberliegenden Seite des Reservoirs liegender, temporär abdeckender und entfernbarer Träger vorgesehen sind.

16. Transdermalsystem nach Anspruch 15, dadurch gekennzeichnet, daß die Abdeckung aus Kunststoffolie besteht.

17. Transdermalsystem nach Anspruch 15 oder 16, dadurch gekennzeichnet, daß der Träger aus silikonisierter Kunststoffolie oder silikonisiertem Papier besteht.

18. Transdermalsystem nach Anspruch 16 oder 17, dadurch gekennzeichnet, daß es sich bei der Kunststoffolie um Polyester-, Polyethylen- oder Polypropylenfolie handelt.

19. Verwendung eines Transdermalsystems nach einem der vorherigen Ansprüche zur Behandlung von Nausea und/oder Emesis.
